# EUROPEAN PATENT SPECIFICATION

(11) **EP 0 376 075 B1**
(45) Date of publication and mention of the grant of the patent: **22.06.1994**
(21) Application number: 89123137.5
(22) Date of filing: 14.12.1989
(51) Int. Cl.: C07C 67/10, C07C 69/76, C07C 69/78, C07C 69/54, C07C 69/44, C07C 69/347, C07C 69/24

(54) **Method for the preparation of an alkenyl carboxylate**
Verfahren zur Herstellung eines Carbonsäurealkenylesters
Méthode de préparation d'un carboxylate d'alcényle

(30) Priority: 26.12.1988 JP 330045/88
(43) Date of publication of application: 04.07.1990
(73) Proprietor: SHIN-ETSU VINYL ACETATE CO., LTD., Sakai-shi Osaka-fu (JP)
(72) Inventor: Honma, Yoshihiro, Sakai-shi Osaka-fu (JP); Kawamoto, Fumiaki, Hashimoto-shi Wakayama-ken (JP); Tanaka, Shozo, Yamatotakada-shi Nara-ken (JP)
(74) Representative: Jaeger, Klaus, Dr.

(56) References cited:
- DE-A- 2 823 660
- GB-A- 1 486 443

## Description

The present invention relates to a method for the preparation of an alkenyl carboxylate or, more particularly, to a method for the preparation of an alkenyl carboxylate by the ester-exchange reaction between a carboxylic acid and an alkenyl carboxylate of less value in the presence of a catalyst having high activity.

It is old that an alkenyl carboxylate, such as a vinyl carboxylate, of high value other than vinyl acetate is prepared by the ester-exchange reaction between a carboxylic acid and a less valuable alkenyl carboxylate, such as vinyl acetate, in the presence of an ester-exchange catalyst. The most traditional catalyst is a combination of mercury acetate and sulfuric acid but the method using such a catalyst is disadvantageous due to the use of a toxic mercury compound and relatively large amount of an ethylidene diester and the like formed as the by-products. Accordingly, this method has later been substituted by a method using a palladium compound as the catalyst as is taught in West German Patent No. 1,127,888, Chemische Berichte, volume 102, page 2939 (1969) and Angewandte Chemie, volume 74, page 93 (1962). In this palladium-catalyzed ester-exchange reaction, the divalent palladium compound is reduced in the course of the reaction to lose the catalytic activity so that the velocity of the reaction is gradually decreased unless an unduly large amount of the palladium compound is used to maintain the reaction velocity at the initial stage resulting in a great industrial disadvantage due to the large costs for the palladium compound. The deactivation of the palladium catalyst can be reduced when a chelate compound of palladium is used as the catalyst as is taught in Tetrahedron, volume 28, page 233 (1972). This method is also not industrially advantageous because chelating agents are generally expensive.

The inventors have previously proposed to conduct the ester-exchange reaction in the presence of palladium acetate and an alkali metal salt of a carboxylic acid (Japanese Patent Kokai 53-77005) or in the presence of a palladium carboxylate and a carbonate, hydrogencarbonate or hydroxide of an alkali metal (Japanese Patent Kokai 53-127410). Although these methods are effective in promoting the catalytic ester-exchange reaction, the problem of deactivation of the palladium compound by reduction is left without complete solution and the reaction velocity is subject to gradual decrease unless a large amount of the palladium compound is used so that the methods have not been practiced industrially due to the economical disadvantage.

The inventors have further continued their investigations to solve the above mentioned problem and arrived at a discovery that deactivation of a palladium catalyst can be prevented by the addition of a copper compound or compounds of which at least one is a halide or, preferably, bromide and an alkali metal, e.g., sodium and potassium, compound to the reaction mixture to exhibit the so-called redox-type catalysis so that the palladium compound is re-oxidized maintaining the activity as is described in Japanese Patent Kokai 54-59203 and 55-104229. This method has been practiced industrially resulting in production of desired alkenyl carboxylates with some success. This method has been subsequently precised by using a divalent copper salt as the copper compound and a combination of a lithium halide and a dialkali or trialkali phosphate as the alkali metal compound (Japanese Patent Kokai 63-39835). Several problems have been left unsolved even in this method that the working efficiency is low because of the difficulty in dissolving sodium or potassium phosphate compound as the alkali metal phosphate in the reaction mixture and the lithium salts are hardly soluble in the reaction mixture resulting in a low conversion of the starting materials due to the poor performance thereof as a cocatalyst.

### SUMMARY OF THE INVENTION

The present invention accordingly has an object to provide an efficient method for the preparation of an alkenyl carboxylate by using a novel and highly active and durable catalyst system for the ester-exchange reaction so as to greatly enhance the conversion of the starting materials.

Thus, the method of the present invention for the preparation of an alkenyl carboxylate comprises heating a liquid mixture of a monobasic carboxylic acid represented by the general formula

RCOOH, (Ia)

in which R is an aliphatic or aromatic monovalent hydrocarbon group, or a polybasic carboxylic acid represented by the general formula

R′(COOH)ₙ, (Ib)

in which n is a positive integer of at least 2 and R′ is an aliphatic or aromatic n-valent hydrocarbon group, and an alkenyl carboxylate represented by the general formula

R¹CO-O-R², (II)

in which R¹ is a hydrogen atom or a monovalent hydrocarbon group different from R or R′ and R² is an alkenyl group, in the presence of a catalyst system comprising:
(a) palladium in an elementary form or a palladium compound;
(b) copper bromide or a combination of a copper compound and an inorganic bromine compound; and
(c) a lithium compound selected from the group consisting of lithium carboxylates, lithium carbonate, lithium hydrogencarbonate and lithium hydroxide,
to effect an ester-exchange reaction forming an alkenyl carboxylate represented by the general formula RCOOR² or R′(COOR²)ₙ, each symbol having the same meaning as defined above.

### DETAILED DESCRIPTION OF THE PREFERRED EMBODIMENTS

As is described above, the ester-exchange reaction of the inventive method is characterized by the use of a specific catalyst system comprising the catalytic ingredients (a), (b) and (c). This unique method of the invention has been established as a result of the investigations on the cocatalytic effect of an alkali metal compound with an object to further enhance the conversion of the starting materials in the ester-exchange reaction. Namely, it was found that the cocatalytic effect of an alkali metal compound depends more on the kind of the alkali metal rather than on the type of the residue or counter ion forming the alkali metal compound with the alkali metal element or ion or, in other words, the reaction velocity obtained by the use of an alkali metal compound depends on the kind of the alkali metal in an increasing order of potassium < sodium < lithium assuming that the compounds are formed with the same counter ion or residue while the types of the counter ion, such as carboxylate ions, carbonate ion, hydrogencarbonate ion, hydroxyl ion, phosphate ion, hydrogenphosphate ion and the like, have no intrinsic influences on the cocatalytic effect of an alkali metal compound although the solubility of the salt in the reaction mixture may be affected thereby.

It was also discovered that, among the lithium compounds having higher cocatalytic effects than the corresponding compounds of sodium or potassium, lithium acetate, lithium carbonate, lithium hydroxide and the like having higher mobility as a lithium ion in the reaction mixture have higher cocatalytic effects than other lithium compounds such as lithium halides and phosphate salts of lithium leading to completion of the present invention.

One of the starting materials in the ester-exchange reaction according to the inventive method is an aliphatic or aromatic monobasic carboxylic acid or polybasic carboxylic acid represented by the general formula (Ia) and (Ib) given above, respectively. The carboxylic acid should be selected, of course, corresponding to the desired alkenyl carboxylate. Examples of the carboxylic acid to which the inventive method is applicable include monobasic aliphatic saturated carboxylic acids such as formic acid, acetic acid, propionic acid, pivalic acid, 2-ethylhexoic acid, caprylic acid, capric acid, lauric acid, stearic acid and the like, monobasic aliphatic unsaturated carboxylic acids such as acrylic acid, methacrylic acid, crotonic acid, sorbic acid, oleic acid and the like, monobasic aromatic saturated carboxylic acids such as benzoic acid, 4-tert-butyl benzoic acid, cinnamic acid and the like, monobasic substituted carboxylic acids such as monochloroacetic acid, 4-chlorobenzoic acid, salicylic acid and the like and polybasic carboxylic acids such as succinic acid, glutaric acid, adipic acid, isophthalic acid, terephthalic acid and the like.

The other starting material to be reacted with the carboxylic acid is an alkenyl carboxylate represented by the general formula (II) given above, of which the carboxyl residue is different from that in the carboxylic acid. Examples of the alkenyl group include vinyl, allyl and isopropenyl groups, though not particularly limitative thereto. Particular examples suitable as the alkenyl carboxylate in the inventive method include vinyl formate, vinyl acetate, vinyl propionate, vinyl lactate, vinyl cyclopropanecarboxylate, isopropenyl acetate allyl acetate and the like, of which relatively inexpensive acetates are usually preferred. The amount of the alkenyl carboxylate as one of the starting materials in the reaction mixture is in the range from 1 to 10 moles per mole of the carboxyl groups in the carboxylic acid as the other starting material although the exact proportion should be adequately selected in consideration of the yield of the product, conditions for the isolation and purification of the product from the reaction mixture and other factors.

The principal ingredient (a) of the catalyst system used in the inventive method is a palladium catalyst which can be in an elementary form, e.g., palladium black, or in the form of a palladium compound including carboxylates such as palladium acetate and the like, inorganic salts such as palladium chloride, palladium bromide and the like and complex compounds of palladium. These catalytic ingredients of palladium can be used in combination of two kinds or more according to need. The amount of the palladium catalyst added to the reaction mixture is in the range from 0.0001 to 0.001 mole as palladium per mole of the carboxylic acid as the starting material.

The catalytic ingredient (b) of the catalyst system, which acts as a redox agent to oxidize the palladium catalyst, in the inventive method is copper bromide or a combination of a copper compound and a bromine compound. It is preferable that the copper in the copper bromide and the copper compound is in the divalent state as in copper (II) bromide. Suitable copper compounds other than copper (II) bromide include compounds of divalent copper such as copper (II) acetate, copper (II) chloride, copper (II) oxide and the like. The bromine compound used in combination with the above mentioned copper compound is exemplified by inorganic bromine compounds or salts such as hydrogen bromide, calcium bromide, magnesium bromide and the like. The amount of the copper compound added to the reaction mixture is in the range from 0.0002 to 0.002 mole per mole of the carboxylic acid as the starting material. The amount of the bromine compound used when the copper compound is not copper (II) bromide is in the range from 0.0004 to 0.004 mole per mole of the carboxylic acid as the starting material.

The third catalytic ingredient (c) of the catalyst system is a lithium compound selected from the group consisting of lithium carboxylates, e.g., lithium acetate, lithium carbonate, lithium hydrogencarbonate and lithium hydroxide. The amount of the lithium compound added to the reaction mixture is usually in the range from 0.005 to 0.05 mole per mole of the carboxylic acid as the starting material.

The ester-exchange reaction of the inventive method is performed by adding the above specified catalytic ingredients to a mixture of the carboxylic acid and the alkenyl carboxylate as the starting reactants and heating the mixture with agitation. The reaction temperature should not exced the boiling point of the alkenyl carboxylate under normal pressure since the reaction is performed usually without pressurization. When the alkenyl carboxylate is vinyl acetate, for example, the reaction is performed at or in the vicinity of 72 to 73°C in consideration of the higher reaction velocity at an elevated temperature although the reaction can proceed at a lower temperature down to room temperature. An excessively high reaction temperature, which may be possible under pressurization of the reaction mixture, is undesirable in view of the stability of the palladium catalyst and eventual decomposition of the alkenyl carboxylate as the product. The exact reaction temperature naturally depends on various factors such as the desired product of alkenyl carboxylate. The ester-exchange reaction of the inventive method usually is complete or reaches near to the equilibrium within 24 hours.

It is optional that the reaction mixture is admixed with an organic solvent such as tetrahydrofuran, acetonitrile, dimethyl formamide and the like with an object to solve a problem relative to the poor miscibility of the carboxylic acid and the alkenyl carboxylate as the starting materials otherwise to disturb smooth proceeding of the liquid-phase ester-exchange reaction.

In the following, examples and comparative examples are given to illustrate the method of the present invention.

### Example 1.

Into a four-necked flask of 1 liter capacity equipped with a stirrer, thermometer and reflux condenser were introduced 413 g of vinyl acetate, 14.2 mg of palladium chloride, 35.7 mg of copper (II) bromide and 1.1 g of lithium acetate to form a mixture which was heated at 70 °C and agitated for several minutes. Thereafter, 160 g of lauric acid were added to the mixture in the flask and the mixture was agitated for 24 hours at 70 °C to effect the ester-exchange reaction. The thus obtained clear, yellowish green liquid was analyzed by the gas chromatography undertaken in the course of or after completion of the reaction to give the results that the conversion of the starting lauric acid into vinyl laurate was 67% after 4 hours, 76% after 8 hours and 85% after 24 hours of the reaction. The reaction mixture was subjected to distillation under reduced pressure to give about 148 g of a fraction boiling at 125 to 130 °C under a pressure of 5 mmHg. This liquid product contained 99% or more of vinyl laurate according to the result of the gas chromatographic analysis.

### Example 2.

The same experimental procedure as in Example 1 was repeated excepting replacement of 35.7 mg of copper (II) bromide with a combination of 29 mg of copper (II) acetate and 28 mg of lithium bromide. The results of the reaction were substantially the same as in Example 1.

### Example 3.

The same experimental procedure as in Example 2 was repeated excepting replacement of 1.1 g of lithium acetate with 1.2 g of lithium carbonate. The results of the reaction were that the conversion of the starting lauric acid into vinyl laurate was 55%, 68% and 85% after 4 hours, 8 hours and 24 hours, respectively, of the reaction.

### Example 4.

The same experimental procedure as in Example 2 was repeated excepting replacement of 1.1 g of lithium acetate with 0.38 g of lithium hydroxide. The results of the reaction were that the conversion of the starting lauric acid into vinyl laurate was 50%, 66% and 84% after 4 hours, 8 hours and 24 hours, respectively, of the reaction.

### Example 5.

The same experimental procedure as in Example 2 was repeated excepting replacement of 28 mg of lithium bromide with 64 mg of calcium bromide. The results of the reaction were that the conversion of the starting lauric acid into vinyl laurate was 60%, 77% and 86% after 4 hours, 8 hours and 24 hours, respectively, of the reaction.

### Example 6.

The same experimental procedure as in Example 2 was repeated excepting replacement of 14.2 mg of palladium chloride with 18 mg of palladium acetate and 28 mg of lithium bromide with 26 mg of hydrogen bromide added as hydrobromic acid containing 47% by weight of hydrogen bromide and decrease of the amount of lithium acetate from 1.1 g to 0.54 g. The results of the reaction were that the conversion of the starting lauric acid into vinyl laurate was 42%, 65% and 83% after 4 hours, 8 hours and 24 hours, respectively, of the reaction.

### Example 7.

The ester-exchange reaction between 98 g of benzoic acid and 413 g of vinyl acetate was conducted substantially in the same manner as in Example 1 excepting replacement of 14.2 mg of palladium chloride with 8.5 mg of palladium black and increase of the amount of copper (II) bromide from 35.7 mg to 71 mg. The results of the reaction were that the conversion of the starting benzoic acid into vinyl benzoate was 47%, 71% and 83% after 4 hours, 8 hours and 24 hours, respectively, of the reaction.

### Example 8.

The ester-exchange reaction between 98 g of benzoic acid and 413 g of vinyl acetate was conducted substantially in the same manner as in Example 2 excepting an increase in the amount of lithium bromide from 28 mg to 56 mg. The results of the reaction were that the conversion of the starting benzoic acid into vinyl benzoate was 40%, 64% and 84% after 4 hours, 8 hours and 24 hours, respectively, of the reaction.

### Example 9.

The same experimental procedure as in Example 8 was repeated excepting replacement of 1.1 g of lithium acetate with 1.2 g of lithium carbonate. The results of the reaction were substantially the same as in Example 8.

### Example 10.

The same experimental procedure as in Example 8 was repeated excepting replacement of 1.1 g of lithium acetate with 0.38 g of lithium hydroxide. The results of the reaction were that the conversion of the starting benzoic acid into vinyl benzoate was 37%, 58% and 83% after 4 hours, 8 hours and 24 hours, respectively, of the reaction.

### Example 11.

The ester-exchange reaction between 59 g of propionic acid and 207 g of vinyl acetate was conducted substantially in the same manner as in Example 1 using a catalyst system composed of 17 mg of palladium chloride, 73 mg of copper (II) acetate, 69 mg of lithium bromide and 1.1 g of lithium acetate. The results of the reaction were that the conversion of the starting propionic acid into vinyl propionate was 60%, 68% and 71% after 4 hours, 8 hours and 24 hours, respectively, of the reaction.

### Example 12.

The same experimental procedure as in Example 11 was repeated excepting replacement of 69 mg of lithium bromide with 74 mg of magnesium bromide. The results of the reaction were that the conversion of the starting propionic acid into vinyl propionate was 51%, 67% and 73% after 4 hours, 8 hours and 24 hours, respectively, of the reaction.

### Example 13.

The ester-exchange reaction between 58 g of acrylic acid and 207 g of vinyl acetate was conducted substantially in the same manner as in Example 1 excepting the use of a catalyst system composed of 63 mg of palladium acetate, 268 mg of copper (II) bromide and 1.1 g of lithium acetate and decrease of the reaction temperature from 70 °C to 40 °C. The results of the reaction were that the conversion of the starting acrylic acid into vinyl acrylate was 32%, 60% and 73% after 4 hours, 8 hours and 24 hours, respectively, of the reaction.

### Example 14.

The ester-exchange reaction between 142 g of 4-tert-butyl benzoic acid and 413 g of vinyl acetate was conducted substantially in the same manner as in Example 1 by using the same catalyst system in the same amounts of the respective catalytic ingredients as in Example 8. The results of the reaction were that the conversion of the starting 4-tert-butyl benzoic acid into vinyl 4-tert-butyl benzoate was 51%, 71% and 86% after 4 hours, 8 hours and 24 hours, respectively, of the reaction.

### Example 15.

The ester-exchange reaction between 82 g of pivalic acid and 207 g of vinyl acetate was conducted substantially in the same manner as in Example 1 excepting the use of a catalyst system composed of 21.3 mg of palladium chloride, 58 mg of copper (II) acetate, 83 mg of lithium bromide and 1.1 g of lithium acetate. The results of the reaction were that the conversion of the starting pivalic acid into vinyl pivalate was 52%, 63% and 71% after 4 hours, 8 hours and 24 hours, respectively, of the reaction.

### Example 16.

The same experimental procedure as in Example 15 was repeated excepting replacement of 1.1 g of lithium acetate with 1.2 g of lithium carbonate. The results of the reaction were that the conversion of the starting pivalic acid into vinyl pivalate was 43%, 57% and 70% after 4 hours, 8 hours and 24 hours, respectively, of the reaction.

### Example 17.

The ester-exchange reaction between 88 g of adipic acid and 517 g of vinyl acetate was conducted substantially in the same manner as in Example 1 excepting the use of a catalyst system composed of 42.6 mg of palladium chloride, 96 mg of copper (II) acetate, 83 mg of lithium bromide, 107 mg of copper (II) bromide and 0.79 g of lithium acetate. The results of the reaction were that the conversion of the starting adipic acid into divinyl adipate was 24%, 52% and 71% after 4 hours, 8 hours and 24 hours, respectively, of the reaction.

### Example 18.

The same experimental procedure as in Example 17 was repeated excepting replacement of 42.6 mg of palladium chloride with 53.9 mg of palladium acetate and omission of copper acetate and lithium bromide. The results of the reaction were that the conversion of the starting adipic acid into divinyl adipate was 29%, 62% and 71% after 4 hours, 8 hours and 24 hours, respectively, of the reaction.

### Comparative Example 1.

The same experimental procedure as in Example 2 was repeated excepting replacement of 1.1 g of lithium acetate with 1.3 g of sodium acetate. The results of the reaction were that the conversion of the starting lauric acid into vinyl laurate was 27%, 33% and 48% after 4 hours, 8 hours and 24 hours, respectively, of the reaction.

### Comparative Example 2.

The same experimental procedure as in Comparative Example 1 was repeated excepting replacement of 1.3 g of sodium acetate with 2.3 g of disodium hydrogenphosphate. A small amount of insoluble matter was found in the reaction mixture. The results of the reaction were that the conversion of the starting lauric acid into vinyl laurate was 21%, 26% and 38% after 4 hours, 8 hours and 24 hours, respectively, of the reaction.

### Comparative Example 3.

The same experimental procedure as in Comparative Example 1 was repeated excepting replacement of 1.3 g of sodium acetate with 1.6 g of potassium acetate. A small amount of insoluble matter was found in the reaction mixture. The results of the reaction were that the conversion of the starting lauric acid into vinyl laurate was 15%, 20% and 33% after 4 hours, 8 hours and 24 hours, respectively, of the reaction.

### Comparative Example 4.

The same experimental procedure as in Example 8 was repeated excepting replacement of 1.1 g of lithium acetate with 1.3 g of sodium acetate. The results of the reaction were that the conversion of the starting benzoic acid into vinyl benzoate was 22%, 34% and 57% after 4 hours, 8 hours and 24 hours, respectively, of the reaction.

### Comparative Example 5.

The same experimental procedure as in Comparative Example 4 was repeated excepting replacement of 1.3 g of sodium acetate with 2.6 g of sodium orthophosphate. The results of the reaction were that the conversion of the starting benzoic acid into vinyl benzoate was 25%, 34% and 56% after 4 hours, 8 hours and 24 hours, respectively, of the reaction.

### Comparative Example 6.

The same experimental procedure as in Comparative Example 4 was repeated excepting replacement of 1.3 g of sodium acetate with 1.2 g of potassium acetate. A somewhat long time was taken in obtaining a clear reaction mixture by dissolving the catalytic ingredients. The results of the reaction were that the conversion of the starting benzoic acid into vinyl benzoate was 15%, 20% and 36% after 4 hours, 8 hours and 24 hours, respectively, of the reaction.

### Comparative Example 7.

The same experimental procedure as in Comparative Example 4 was repeated excepting replacement of 1.3 g of sodium acetate with 3.4 g of potassium orthophosphate. A small amount of insoluble matter was found in the reaction mixture. The results of the reaction were that the conversion of the starting benzoic acid into vinyl benzoate was 15%, 19% and 34% after 4 hours, 8 hours and 24 hours, respectively, of the reaction.

### Comparative Example 8.

The same experimental procedure as in Example 11 was repeated excepting replacement of 1.1 g of lithium acetate with 1.3 g of sodium acetate. The results of the reaction were that the conversion of the starting propionic acid into vinyl propionate was 35%, 45% and 61% after 4 hours, 8 hours and 24 hours, respectively, of the reaction.

### Comparative Example 9.

The same experimental procedure as in Comparative Example 8 was repeated excepting replacement of 1.3 of sodium acetate with 2.3 g of disodium hydrogenphosphate. White precipitates were found in the reaction mixture. The results of the reaction were that the conversion of the starting propionic acid into vinyl propionate was 26%, 34% and 53% after 4 hours, 8 hours and 24 hours, respectively, of the reaction.

### Comparative Example 10.

The same experimental procedure as in Comparative Example 8 was repeated excepting replacement of 1.3 g of sodium acetate with 1.6 g of potassium acetate. The results of the reaction were that the conversion of the starting propionic acid into vinyl propionate was 27%, 39% and 59% after 4 hours, 8 hours and 24 hours, respectively, of the reaction.

### Comparative Example 11.

The same experimental procedure as in Example 13 was repeated excepting replacement of 1.1 g of lithium acetate with 1.3 g of sodium acetate. The results of the reaction were that the conversion of the starting acrylic acid into vinyl acrylate was 6%, 14% and 42% after 4 hours, 8 hours and 24 hours, respectively, of the reaction.

### Comparative Example 12.

The same experimental procedure as in Comparative Example 11 was repeated excepting replacement of 1.3 g of sodium acetate with 2.6 g of sodium orthophosphate. The results of the reaction were that the conversion of the starting acrylic acid into vinyl acrylate was 13%, 22% and 51% after 4 hours, 8 hours and 24 hours, respectively, of the reaction.

### Comparative Example 13.

The same experimental procedure as in Comparative Example 11 was repeated excepting replacement of 1.3 g of sodium acetate with 1.6 g of potassium acetate. The results of the reaction were that the conversion of the starting acrylic acid into vinyl acrylate was 11%, 16% and 23% after 4 hours, 8 hours and 24 hours, respectively, of the reaction showing relatively rapid deactivation of the catalyst with the conversion levelling off.

### Comparative Example 14.

The same experimental procedure as in Example 14 was repeated excepting replacement of 1.1 g of lithium acetate with 1.3 g of sodium acetate. The results of the reaction were that the conversion of the starting 4-tert-butyl benzoic acid into vinyl 4-tert-butyl benzoate was 26%, 35% and 56% after 4 hours, 8 hours and 24 hours, respectively, of the reaction.

### Comparative Example 15.

The same experimental procedure as in Comparative Example 14 was repeated excepting replacement of 1.3 g of sodium acetate with 2.6 g of sodium orthophosphate. The results of the reaction were substantially the same as in Comparative Example 14.

### Comparative Example 16.

The same experimental procedure as in Comparative Example 14 was repeated excepting replacement of 1.3 g of sodium acetate with 1.6 g of potassium acetate. The solubility behavior of the catalytic ingredients was poor in the reaction mixture. The results of the reaction were that the conversion of the starting 4-tert-butyl benzoic acid into vinyl 4-tert-butyl benzoate was 13%, 17% and 33% after 4 hours, 8 hours and 24 hours, respectively, of the reaction.

### Comparative Example 17.

The same experimental procedure as in Comparative Example 14 was repeated excepting replacement of 1.3 g of sodium acetate with 2.8 g of dipotassium hydrogenphosphate. A considerable amount of the catalytic ingredients remained undissolved in the reaction mixture. The results of the reaction were that the conversion of the starting 4-tert-butyl benzoic acid into vinyl 4-tert-butyl benzoate was 12%, 16% and 28% after 4 hours, 8 hours and 24 hours, respectively, of the reaction.

### Comparative Example 18.

The same experimental procedure as in Example 15 was repeated excepting replacement of 1.1 g of lithium acetate with 1.3 g of sodium acetate. The results of the reaction were that the conversion of the starting pivalic acid into vinyl pivalate was 21%, 30% and 49% after 4 hours, 8 hours and 24 hours, respectively, of the reaction.

### Comparative Example 19.

The same experimental procedure as in Comparative Example 18 was repeated excepting replacement of 1.3 g of sodium acetate with 2.6 g of sodium orthophosphate. The results of the reaction were that the conversion of the starting pivalic acid into vinyl pivalate was 21%, 27% and 45% after 4 hours, 8 hours and 24 hours, respectively, of the reaction.

### Comparative Example 20.

The same experimental procedure as in Comparative Example 18 was repeated excepting replacement of 1.3 g of sodium acetate with 1.6 g of potassium acetate. The results of the reaction were that the conversion of the starting pivalic acid into vinyl pivalate was 8%, 10% and 15% after 4 hours, 8 hours and 24 hours, respectively, of the reaction showing rapid deactivation of the catalyst with the conversion levelling off.

### Comparative Example 21.

The same experimental procedure as in Comparative Example 4 was repeated excepting replacement of 1.3 g of sodium acetate with 1.4 g of lithium bromide so that the overall amount of the lithium bromide was 1.456 g. The results of the reaction were that the conversion of the starting benzoic acid into vinyl benzoate was only 0.1%, 0.2% and 0.7% after 4 hours, 8 hours and 24 hours, respectively, of the reaction showing that the catalyst system had almost no activity.

### Comparative Example 22.

The same experimental procedure as in Comparative Example 21 was repeated excepting replacement of 1.4 g of lithium bromide with 0.7 g of lithium chloride. The results of the reaction were that the conversion of the starting benzoic acid into vinyl benzoate was only 0.5%, 1.0% and 6.5% after 4 hours, 8 hours and 24 hours, respectively, of the reaction.

### Comparative Example 23.

The same experimental procedure as in Comparative Example 1 was repeated excepting replacement of 1.3 g of sodium acetate with 1.9 g of lithium orthophosphate, which was hardly soluble in the reaction mixture. The results of the reaction were that the conversion of the starting lauric acid into vinyl laurate was only 8%, 16% and 25% after 4 hours, 8 hours and 24 hours, respectively, of the reaction.

### Comparative Example 24.

The same experimental procedure as in Comparative Example 21 was repeated excepting replacement of 1.4 g of lithium bromide with 1.9 g of lithium orthophosphate, which was hardly soluble in the reaction mixture. The results of the reaction were that the conversion of the starting benzoic acid into vinyl benzoate was only 2%, 4% and 17% after 4 hours, 8 hours and 24 hours, respectively, of the reaction.

### Comparative Example 25.

The same experimental procedure as in Comparative Example 11 was repeated excepting replacement of 1.3 g of sodium acetate with 1.9 g of lithium orthophosphate, which was hardly soluble in the reaction mixture. The results of the reaction were that the conversion of the starting acrylic acid into vinyl acrylate was only 1%, 2% and 9% after 4 hours, 8 hours and 24 hours, respectively, of the reaction.

### Comparative Example 26.

The same experimental procedure as in Example 14 was repeated excepting replacement of 1.1 g of lithium acetate with 1.9 g of lithium orthophosphate, which was hardly soluble in the reaction mixture, and decrease of the amount of lithium bromide from 56 mg to 28 mg. The results of the reaction were that the conversion of the starting 4-tert-butyl benzoic acid into vinyl 4-tert-butyl benzoate was 4%, 8% and 20% after 4 hours, 8 hours and 24 hours, respectively, of the reaction.

### Comparative Example 27.

The same experimental procedure as in Example 17 was repeated excepting decrease of the amount of vinyl acetate from 517 g to 413 g and replacement of 0.79 g of lithium acetate with 0.98 g of sodium acetate. The results of the reaction were that the conversion of the starting adipic acid into divinyl adipate was 20%, 32% and 61% after 4 hours, 8 hours and 24 hours, respectively, of the reaction.

### Comparative Example 28.

The same experimental procedure as in Comparative Example 27 was repeated excepting replacement of 0.98 g of sodium acetate with 2.0 g of sodium orthophosphate. The results of the reaction were that the conversion of the starting adipic acid into divinyl adipate was 17%, 29% and 57% after 4 hours, 8 hours and 24 hours, respectively, of the reaction.

### Comparative Example 29.

The same experimental procedure as in Comparative Example 27 was repeated excepting replacement of 0.98 g of sodium acetate with 2.1 g of dipotassium hydrogenphosphate. A considerable amount of insoluble matter was found in the reaction mixture. The results of the reaction were that the conversion of the starting adipic acid into divinyl adipate was 3%, 4% and 5% after 4 hours, 8 hours and 24 hours, respectively, of the reaction.

The above described experimental results in Examples and Comparative Examples indicate that the ester-exchange reaction proceeds nearly to equilibrium within 24 hours when a lithium compound such as lithium acetate, lithium carbonate, lithium hydroxide and the like is used as a cocatalyst, that elementary palladium or a palladium compound can be used as the palladium ingredient of the catalyst system and the effect of redox reaction can be obtained by the use of copper (II) bromide alone or a combination of copper (II) acetate and a bromine compound such as lithium bromide, calcium bromide, magnesium bromide, hydrogen bromide and the like.

It is also evident that the cocatalytic activity exhibited by the lithium compound is specific to the particular lithium compounds and replacement thereof with a lithium halide results in an extremely low conversion of the starting reactants while the cocatalytic activity of lithium phosphate is very low, presumably, due to the low solubility thereof in the reaction mixture.

## Claims

1. A method for the preparation of an alkenyl carboxylate by the ester-exchange reaction between a carboxylic acid and a second alkenyl carboxylate which comprises heating a liquid mixture of a monobasic carboxylic acid represented by the general formula
RCOOH,
in which R is an aliphatic or aromatic monovalent hydrocarbon group, or a polybasic carboxylic acid represented by the general formula
R′(COOH)ₙ,
in which n is a positive integer of at least 2 and R′ is an aliphatic or aromatic n-valent hydrocarbon group, and an alkenyl carboxylate represented by the general formula
R¹CO-O-R²,
in which R¹ is a hydrogen atom or a monovalent hydrocarbon group different from R and R′ and R² is an alkenyl group, in the presence of a catalyst system comprising:
(a) palladium in an elementary form or a palladium compound;
(b) copper bromide or a combination of a copper compound other than copper bromide and an inorganic bromine compound; and
(c) a lithium compound selected from the group consisting of lithium carboxylates, lithium carbonate, lithium hydrogencarbonate and lithium hydroxide,
to effect an ester-exchange reaction forming an alkenyl carboxylate represented by the general formula RCOOR² or R′(COOR²)ₙ, each symbol having the same meaning as defined above.

2. The method for the preparation of an alkenyl carboxylate as claimed in claim 1 wherein the palladium compound is selected from the group consisting of palladium acetate, palladium chloride, palladium bromide and complex compounds of palladium.

3. The method for the preparation of an alkenyl carboxylate as claimed in claim 1 wherein the amount of palladium in the elementary form or the palladium compound is in the range from 0.0001 to 0.001 mole as palladium per mole of the carboxylic acid.

4. The method for the preparation of an alkenyl carboxylate as claimed in claim 1 wherein the copper bromide is copper (II) bromide.

5. The method for the preparation of an alkenyl carboxylate as claimed in claim 1 wherein the copper compound other than the copper bromide is copper (II) acetate, copper (II) chloride or copper (II) oxide.

6. The method for the preparation of an alkenyl carboxylate as claimed in claim 1 wherein the inorganic bromine compound is selected from the group consisting of lithium bromide, hydrogen bromide, calcium bromide and magnesium bromide.

7. The method for the preparation of an alkenyl carboxylate as claimed in claim 1 wherein the amount of the copper bromide or the copper compound is in the range from 0.0002 to 0.002 mole per mole of the carboxylic acid.

8. The method for the preparation of an alkenyl carboxylate as claimed in claim 1 wherein the amount of the inorganic bromine compound combined with the copper compound other than copper bromide is in the range from 0.0004 to 0.004 mole per mole of the carboxylic acid.

9. The method for the preparation of an alkenyl carboxylate as claimed in claim 1 wherein the amount of the lithium compound is in the range from 0.005 to 0.05 mole per mole of the carboxylic acid.

10. The method for the preparation of an alkenyl carboxylate as claimed in claim 1 wherein the amount of the alkenyl carboxylate represented by the general formula R¹CO-O-R² is in the range from 1 to 10 moles per mole of the carboxylic acid.

11. The method for the preparation of an alkenyl carboxylate as claimed in claim 1 wherein the alkenyl carboxylate represented by the general formula R¹CO-O-R² is vinyl acetate.

## Patentansprüche

1. Verfahren zur Herstellung eines Alkenylcarboxylats durch die Umesterungsreaktion zwischen einer Carbonsäure und einem zweiten Alkenylcarboxylat, wobei ein flüssiges Gemisch aus einer monobasischen Carbonsäure der folgenden allgemeinen Formel
RCOOH,
worin R eine aliphatische oder aromatische, monovalente Kohlenwasserstoffgruppe bedeutet,
oder einer polybasischen Carbonsäure der folgenden allgemeinen Formel
R'(COOH)ₙ,
worin n eine positive ganze Zahl von mindestens 2 und R' eine aliphatische oder aromatische, n-valente Kohlenwasserstoffgruppe bedeuten,
und einem Alkenylcarboxylat der folgenden allgemeinen Formel
R¹CO-O-R²,
worin R¹ ein Wasserstoffatom oder eine monovalente Kohlenwasserstoffgruppe, die sich von R und R' unterscheidet, bedeutet und R² eine Alkenylgruppe bedeutet, in Anwesenheit eines Katalysatorsystems aus:
(a) Palladium in elementarer Form oder einer Palladiumverbindung,
(b) Kupferbromid oder einer Kombination aus einer sich von Kupferbromid unterscheidenden Kupferverbindung und einer anorganischen Bromverbindung und
(c) einer Lithiumverbindung, die ausgewählt ist aus der Gruppe bestehend aus Lithiumcarboxylaten, Lithiumcarbonat, Lithiumhydrogencarbonat und Lithiumhydroxid,
zur Durchführung einer Umesterungsreaktion unter Bildung eines Alkenylcarboxylats der folgenden allgemeinen Formel RCOOR² oder R' (COOR² )ₙ, wobei jedes Symbol die oben aufgeführten Bedeutungen besitzt, erhitzt wird.

2. Verfahren zur Herstellung eines Alkenylcarboxylats nach Anspruch 1, bei dem die Palladiumverbindung ausgewählt ist aus der Gruppe bestehend aus Palladiumacetat, Palladiumchlorid, Palladiumbromid und Komplexverbindungen von Palladium.

3. Verfahren zur Herstellung eines Alkenylcarboxylats nach Anspruch 1, bei dem die Menge an Palladium in elementarer Form oder der Palladiumverbindung 0,0001 bis 0,001 Mol an Palladium pro Mol an Carbonsäure beträgt.

4. Verfahren zur Herstellung eines Alkenylcarboxylats nach Anspruch 1, bei dem es sich bei dem Kupferbromid um Kupfer(II)-bromid handelt.

5. Verfahren zur Herstellung eines Alkenylcarboxylats nach Anspruch 1, bei dem es sich bei der Kupferverbindung, die sich von Kupferbromid unterscheidet, um Kupfer(II)-acetat, Kupfer(II)-chlorid oder Kupfer(II)-oxid handelt.

6. Verfahren zur Herstellung eines Alkenylcarboxylats nach Anspruch 1, bei dem die anorganische Bromverbindung ausgewählt ist aus der Gruppe bestehend aus Lithiumbromid, Bromwasserstoff, Kalziumbromid und Magnesiumbromid.

7. Verfahren zur Herstellung eines Alkenylcarboxylats nach Anspruch 1, bei dem die Menge an Kupferbromid oder an Kupferverbindung 0,0002 bis 0,002 Mol pro Mol an Carbonsäure beträgt.

8. Verfahren zur Herstellung eines Alkenylcarboxylats nach Anspruch 1, bei dem die Menge an anorganischer Bromverbindungen in Kombination mit der Kupferverbindung, die sich von Kupferbromid unterscheidet, 0,0004 bis 0,004 Mol pro Mol der Carbonsäure beträgt.

9. Verfahren zur Herstellung eines Alkenylcarboxylats nach Anspruch 1, bei dem die Menge an Lithiumverbindung 0,005 bis 0,05 Mol pro Mol an Carbonsäure beträgt.

10. Verfahren zur Herstellung eines Alkenylcarboxylats nach Anspruch 1, bei dem die Menge an Alkenylcarboxylat der allgemeinen Formel R¹CO-O-R² 1 bis 10 Mol pro Mol der Carbonsäure beträgt.

11. Verfahren zur Herstellung eines Alkenylcarboxylats nach Anspruch 1, bei dem das Alkenylcarboxylat der allgemeinen Formel R¹CO-O-R² Vinylacetat ist.

## Revendications

1. Procédé pour la préparation d'un carboxylate d'alcényle par la réaction d'échange d'ester entre un acide carboxylique et un deuxième carboxylate d'alcényle qui consiste à chauffer un mélange liquide d'un monoacide carboxylique représenté par la formule générale
RCOOH,
dans laquelle R est un groupe hydrocarboné monovalent aromatique ou aliphatique, ou un acide carboxylique polybasique représenté par la formule générale
R'(COOH)ₙ,
dans laquelle n est un nombre entier positif d'au moins 2 et R' est un groupe hydrocarboné n-valent aromatique ou aliphatique, et un carboxylate d'alcényle représenté par la formule générale
R¹CO-O-R²,
dans laquelle R¹ est un atome d'hydrogène ou un groupe hydrocarboné monovalent différent de R et R', et R² est un groupe alcényle, en présence d'un système catalytique comprenant:
(a) du palladium sous une forme élémentaire ou un composé de palladium;
(b) du bromure de cuivre ou une combinaison d'un composé de cuivre autre que le bromure de cuivre et un composé minéral de brome; et
(c) un composé de lithium choisi dans le groupe formé par les carboxylates de lithium, le carbonate de lithium, l'hydrogénocarbonate de lithium et l'hydroxyde de lithium,
pour effectuer une réaction d'échange d'ester formant un carboxylate d'alcényle représenté par la formule générale RCOOR² ou R'(COOR²)ₙ, chaque symbole ayant la même signification que celle définie ci-dessus.

2. Procédé pour la préparation d'un carboxylate d'alcényle selon la revendication 1, dans lequel le composé de palladium est choisi dans le groupe formé par l'acétate de palladium, le chlorure de palladium, le bromure de palladium et les composés complexes du palladium.

3. Procédé pour la préparation d'un carboxylate d'alcényle selon la revendication 1, dans lequel la quantité de palladium sous forme élémentaire ou du composé de palladium est comprise entre 0,0001 à 0,001 mole exprimée en palladium par mole d'acide carboxylique.

4. Procédé pour la préparation d'un carboxylate d'alcényle selon la revendication 1, dans lequel le bromure de cuivre est le bromure de cuivre (II).

5. Procédé pour la préparation d'un carboxylate d'alcényle selon la revendication 1, dans lequel le composé cuivre autre que le bromure de cuivre est l'acétate de cuivre (II), le chlorure de cuivre (II) ou l'oxyde de cuivre (II).

6. Procédé pour la préparation d'un carboxylate d'alcényle selon la revendication 1, dans lequel le composé minéral de brome est choisi dans le groupe formé par le bromure de lithium, le bromure d'hydrogène, le bromure de calcium et le bromure de magnésium.

7. Procédé pour la préparation d'un carboxylate d'alcényle selon la revendication 1, dans lequel la quantité du bromure de cuivre ou du composé cuivre est comprise entre 0,0002 et 0,002 mole par mole d'acide carboxylique.

8. Procédé pour la préparation d'un carboxylate d'alcényle selon la revendication 1, dans lequel la quantité du composé minéral de brome combiné avec le composé du cuivre autre que le bromure de cuivre est comprise entre 0,0004 et 0,004 mole par mole d'acide carboxylique.

9. Procédé pour la préparation d'un carboxylate d'alcényle selon la revendication 1, dans lequel la quantité du composé de lithium est comprise entre 0,005 et 0,05 mole par mole d'acide carboxylique.

10. Procédé pour la préparation d'un carboxylate d'alcényle selon la revendication 1, dans lequel la quantité du carboxylate d'alcényle représenté par la formule générale R¹CO-O-R² est comprise entre 1 et 10 moles par mole d'acide carboxylique.

11. Procédé pour la préparation d'un carboxylate d'alcényle selon la revendication 1, dans lequel le carboxylate d'alcényle représenté par la formule générale R¹CO-O-R² est l'acétate de vinyle.
